# EUROPEAN PATENT APPLICATION

(11) **EP 4 082 533 A1**
(43) Date of publication of application: **02.11.2022**
(21) Application number: 20904510.3
(22) Date of filing: 22.12.2020
(51) Int. Cl.: A61K 9/20, A61K 31/519, C07D 487/04, A61K 47/38, A61P 19/02, A61P 29/00

(54) **JAK KINASE INHIBITOR PHARMACEUTICAL COMPOSITION**

(30) Priority: 23.12.2019 CN 201911334601
(71) Applicant: Jiangsu Hengrui Medicine Co., Ltd., Lianyungang, Jiangsu 222047 (CN)
(72) Inventor: XI, Honglei, Lianyungang, Jiangsu 222047 (CN); JIANG, Qiudong, Lianyungang, Jiangsu 222047 (CN); CHEN, Hao, Lianyungang, Jiangsu 222047 (CN)
(74) Representative: dompatent von Kreisler Selting Werner - Partnerschaft von Patent- und Rechtsanwälten mbB
(86) International application number: PCT/CN2020/138253
(87) International publication number: WO 2021/129600

(57) **Abstract**

A JAK kinase inhibitor pharmaceutical composition, containing (3a*R*,5*s*,6a*S*)-*N*-(3-methoxy-1,2,4-thiadiazol-5-yl)-5-(methyl(7*H*-pyrrolo[2,3-*d*]pyrimidin-4-yl) amino)hexahydrocyclopenta[*c*]pyrrole-2(1*H*)-carboxamide or a pharmaceutically acceptable salt thereof and a co-processed excipient, such as cellulose-lactose. The present invention has good stability, dissolution and bioavailability, and the preparation process thereof is simple and convenient.

## Description

The present application claims priority to Chinese Patent Application No. CN201911334601.5 filed on Dec. 23, 2019, which is incorporated herein by reference in its entirety.

### TECHNICAL FIELD

The disclosure relates to the field of pharmaceutics, and provides a JAK kinase inhibitor pharmaceutical composition comprising (3a*R*,5*s*,6a*S*)-*N*-(3-methoxy-1,2,4-thiadiazol-5-yl)-5-(methyl(7*H*-pyrrolo[2,3-*d*]pyrimidin-4-yl) amino)hexahydrocyclopenta[*c*]pyrrole-2(1*H*)-carboxamide or a pharmaceutically acceptable salt thereof and a co-processed excipient such as cellulose-lactose.

### BACKGROUND

JAK inhibitors can selectively inhibit the JAK kinase and block the JAK/STAT signaling pathway. JAK inhibitors are mainly used for screening therapeutic drugs for diseases of the blood system, tumors, rheumatoid arthritis, psoriasis and the like in clinic. Arthritis is the most common chronic disease worldwide, with about 355 million people suffering from it worldwide, including 100 million or more in China.

Currently, several JAK inhibitors have been in clinical research, such as ruxolitinib for the treatment of the blood disease and tofacitinib for the treatment of rheumatoid arthritis which have been approved by the FDA, both administered orally.

(3a*R*,5*s*,6a*S*)-*N*-(3-methoxy-1,2,4-thiadiazol-5-yl)-5-(methyl(7*H*-pyrrolo[2,3-*d*]pyrimidin-4-yl) amino)hexahydrocyclopenta[*c*]pyrrole-2(1*H*)-carboxamide is a JAK kinase inhibitor compound with *in vivo* and *in vitro* activity and high absorption. CN201780001376.6 describes a composition comprising (3a*R*,5*s*,6a*S*)-*N*-(3-methoxy-1,2,4-thiadiazol-5-yl)-5-(methyl(7*H*-pyrrolo[2,3-*d*]pyrimidin-4-yl) amino)hexahydrocyclopenta[*c*]pyrrole-2(1*H*)-carboxamide and a cellulose ether, and it is believed that the selection of binder directly affects the storage stability of the composition, and that a composition with excellent stability and dissolution rate is obtained using hydroxypropyl methylcellulose. And, the result show that the resulting composition (such as a tablet) after the long-term storage of the above compound has undesirable properties, such as slow dissolution rate and incomplete dissolution.

In another aspect, there are many factors affecting the quality of drug products, which can be summarized into two types, i.e., internal and external factors. The internal and external factors affecting the production quality of drug products generally include four aspects: 1. packaging damage during the transportation of drug products; 2. the climate conditions like temperature and humidity; 3. the temperature and humidity during the transportation; and 4. irregular storage by the pharmaceutical trading enterprises or patients. Considering that the dissolution of the above formulation formula is slower after the storage, there is an urgent need for the technical staff to develop a new formulation formula that meets the product quality requirement, so as to meet the safety requirement of the product for marketing.

### SUMMARY

The disclosure provides a pharmaceutical composition comprising an active ingredient (3a*R*,5*s*,6a*S*)-*N*-(3-methoxy-1,2,4-thiadiazol-5-yl)-5-(methyl(7*H*-pyrrolo[2,3-*d*]pyrimidin-4-yl) amino)hexahydrocyclopenta[*c*]pyrrole-2(1*H*)-carboxamide or a pharmaceutically acceptable salt thereof and a filler, wherein the filler is selected from the group consisting of a co-processed excipient.

The co-processed excipient is an excipient that is prepared by pre-mixing several (a single) excipients and can be directly applied, for example, a marketed lactose compound Cellactose^{®}80 (abbreviated as C80), which is an integrated spherical particle (cellulose is uniformly distributed in the spherical particle) obtained by fully suspending cellulose in a lactose solution and spray drying, and consists of 75% lactose monohydrate and 25% powdered cellulose.

In some embodiments, the co-processed excipient is selected from the group consisting of microcrystalline cellulose-lactose, cellulose-lactose and corn starch-lactose, preferably microcrystalline cellulose-lactose or cellulose-lactose.

In some embodiments, the microcrystalline cellulose-lactose refers to a spray-dried mixture of lactose and microcrystalline cellulose, for example, includes a spray-dried mixture comprising about 75 wt% lactose and about 25 wt% microcrystalline cellulose, such as, but not limited to, microcrystalline cellulose-lactose (microcellac 100) sold by the Meggle corporation.

In some embodiments, the cellulose-lactose refers to a spray-dried mixture of lactose and cellulose, for example, includes a spray-dried mixture comprising about 75 wt% lactose and about 25 wt% cellulose, such as, but not limited to, cellulose-lactose (abbreviated as C80) sold by the Meggle corporation.

In some embodiments, the cellulose-lactose has a bulk density of about 370 g/L.

In some embodiments, the cellulose-lactose has a bulk density of about 490 g/L.

In some embodiments, the cellulose-lactose has a Carr index of about 24.49%.

In some embodiments, the active ingredient in the pharmaceutical composition, based on the total weight of the pharmaceutical composition, has a content of about 0.1%-30%, specifically about 0.1%, 0.2%, 0.3%, 0.4%, 0.5%, 0.6%, 0.7%, 0.8%, 0.9%, 1.0%, 1.5%, 2.0%, 2.5%, 3.0%, 3.5%, 4.0%, 4.5%, 5.0%, 5.5%, 6.0%, 6.5%, 7.0%, 7.5%, 8.0%, 8.5%, 9.0%, 9.5%, 10.0%, 10.5%, 11.0%, 11.5%, 12.0%, 12.5%, 13.0%, 13.5%, 14.0%, 14.5%, 15.0%, 15.5%, 16.0%, 16.5%, 17.0%, 17.5%, 18.0%, 18.5%, 19.0%, 19.5%, 20.0%, 20.5%, 21.0%, 21.5%, 22.0%, 22.5%, 23.0%, 23.5%, 24.0%, 24.5%, 25.0%, 25.5%, 26.0%, 26.5%, 27.0%, 27.5%, 28.0%, 28.5%, 29.0%, 29.5%, 30.0% or any value between any two of the values, preferably 1.0%-15.0%.

In another aspect, the filler in the pharmaceutical composition provided in some embodiments, based on the total weight of the pharmaceutical composition, has a content of about 40%-95%, specifically about 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95% or any value between any two of the values, preferably 60%-92%.

The pharmaceutical composition provided in some other embodiments further comprises a disintegrant, wherein the disintegrant, based on the total weight of the pharmaceutical composition, has a content of about 1%-30%, specifically about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30% or any value between any two of the values, preferably 2%-20%.

In some embodiments, the disintegrant is selected from the group consisting of, but not limited to, at least one of pre-gelatinized starch, adipic acid, alginic acid, gelatinized starch, crospolyvinylpyrrolidone and low-substituted hydroxypropylcellulose.

In some embodiments, the disintegrant is free of a metal element, wherein the metal element may be an alkali metal or alkaline earth metal element or aluminum.

The pharmaceutical composition provided in some embodiments further comprises at least one of a glidant and a lubricant, wherein the glidant or the lubricant, based on the total weight of the pharmaceutical composition, has a content of about 0.5%-5%, specifically about 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9%, 5.0% or any value between any two of the values, preferably about 1%-5%.

In some embodiments, the lubricant is selected from the group consisting of, but not limited to, at least one of stearic acid, glyceryl behenate, hydrogenated vegetable oil and silicon dioxide. In some embodiments, the lubricant is free of a metal element, wherein the metal element may be an alkali metal or alkaline earth metal element or aluminum.

In another aspect, in some embodiments, the glidant may be at least one of silicon dioxide, light anhydrous silicic acid, crystalline cellulose, stearic acid and corn starch, preferably silicon dioxide.

In some embodiments, the glidant is free of a metal element, wherein the metal element may be an alkali metal or alkaline earth metal element or aluminum.

In another aspect, the filler of the pharmaceutical composition of the disclosure further comprises lactose.

In some embodiments, the co-processed excipient and the lactose in the filler are in a weight ratio of about 1:2-5:1, specifically 1:2, 1:1.5, 1:1, 1.5:1, 2:1 or any value between any two of the values, preferably 1:2-2:1.

In some embodiments, the pharmaceutical composition comprises the following ingredients based on the total weight of the pharmaceutical composition:
1) 0.1%-30% (3a*R*,5*s*,6a*S*)-*N*-(3-methoxy-1,2,4-thiadiazol-5-yl)-5-(methyl(7*H*-pyrrolo[2,3-*d*]pyrimidin-4-yl)a mino)hexahydrocyclopenta[*c*]pyrrole-2(1*H*)-carboxamide or a pharmaceutically acceptable salt thereof,
2) 40%-95% filler, wherein the co-processed excipient and the lactose are in a weight ratio of 1:2-5:1,
3) optionally 1%-30% disintegrant,
4) optionally 0.5%-5% glidant, and
5) optionally 0.5%-5% lubricant,
wherein further, the pharmaceutical composition is free of an excipient containing a metal element.

The disclosure also provides a pharmaceutical composition comprising (3a*R*,5*s*,6a*S*)-*N*-(3-methoxy-1,2,4-thiadiazol-5-yl)-5-(methyl(7*H*-pyrrolo[2,3-*d*]pyrimidin-4-yl) amino)hexahydrocyclopenta[*c*]pyrrole-2(1*H*)-carboxamide or a pharmaceutically acceptable salt thereof, wherein the pharmaceutical composition is free of an excipient containing a metal element. Further, the pharmaceutical composition optionally comprises at least one of a filler, a disintegrant, a glidant and a lubricant. In some embodiments, the filler is selected from the group consisting of at least one of lactose, microcrystalline cellulose-lactose, cellulose-lactose and corn starch-lactose.

In other embodiments, the filler is selected from the group consisting of lactose and microcrystalline cellulose-lactose or cellulose-lactose. Further, microcrystalline cellulose-lactose or cellulose-lactose and lactose are in a weight ratio of 1:2-5:1, e.g., 1:2, 1:1.5, 1:1, 1.5:1, 2:1 or any value between any two of the values.

In some embodiments, the disintegrant is selected from the group consisting of, but not limited to, at least one of pre-gelatinized starch, adipic acid, alginic acid, gelatinized starch, crospolyvinylpyrrolidone and low-substituted hydroxypropylcellulose. Further, the disintegrant, based on the total weight of the pharmaceutical composition, has a content of about 1%-30%, specifically about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30% or any value between any two of the values, preferably 2%-20%.

In some embodiments, the lubricant is selected from the group consisting of, but not limited to, at least one of stearic acid, glyceryl behenate, hydrogenated vegetable oil and silicon dioxide. In another aspect, in some embodiments, the glidant may be at least one of silicon dioxide, light anhydrous silicic acid, crystalline cellulose, stearic acid and corn starch, preferably silicon dioxide.

In some embodiments, the glidant or the lubricant, based on the total weight of the pharmaceutical composition, has a content of about 0.5%-5%, specifically about 0.5%, 0.6%, 0.7%, 0.8%, 0.9%, 1.0%, 1.1%, 1.2%, 1.3%, 1.4%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9%, 5.0% or any value between any two of the values, preferably 1%-5%.

In some embodiments, the pharmaceutical composition comprises the following ingredients based on the total weight of the pharmaceutical composition:
1) 0.1%-30% (3a*R*,5*s*,6a*S*)-*N*-(3-methoxy-1,2,4-thiadiazol-5-yl)-5-(methyl(7*H*-pyrrolo[2,3-*d*]pyrimidin-4-yl)a mino)hexahydrocyclopenta[*c*]pyrrole-2(1*H*)-carboxamide or a pharmaceutically acceptable salt thereof, wherein the pharmaceutical composition is free of an excipient containing a metal element,
2) optionally 40%-95% filler,
3) optionally 1%-30% disintegrant,
4) optionally 0.5%-5% glidant, and
5) optionally 0.5%-5% lubricant.

The pharmaceutical composition provided in some embodiments comprises the following ingredients based on the total weight of the pharmaceutical composition:
1) 0.1%-30% (3a*R*,5*s*,6a*S*)-*N*-(3-methoxy-1,2,4-thiadiazol-5-yl)-5-(methyl(7*H*-pyrrolo[2,3-*d*]pyrimidin-4-yl) amino)hexahydrocyclopenta[*c*]pyrrole-2(1*H*)-carboxamide or a pharmaceutically acceptable salt thereof, wherein the pharmaceutical composition is free of an excipient containing a metal element,
2) 40%-95% filler containing a co-processed excipient, wherein the co-processed excipient is preferably microcrystalline cellulose-lactose, cellulose-lactose or corn starch-lactose,
3) 1%-30% disintegrant,
4) optionally 0.5%-5% glidant, and
5) optionally 0.5%-5% lubricant.

The active ingredient (3a*R*,5*s*,6a*S*)-*N*-(3-methoxy-1,2,4-thiadiazol-5-yl)-5-(methyl(7*H*-pyrrolo[2,3-*d*]pyrimidin-4-yl) amino)hexahydrocyclopenta[*c*]pyrrole-2(1*H*)-carboxamide or a pharmaceutically acceptable salt thereof in the pharmaceutical composition provided in another aspect of the disclosure has a particle size of 90% (abbreviated as D90) of no more than about 50 µm, e.g., 10 µm, 11 µm, 12 µm, 13 µm, 14 µm, 15 µm, 16 µm, 17 µm, 18 µm, 19 µm, 20 µm, 21 µm, 22 µm, 23 µm, 24 µm, 25 µm, 26 µm, 27 µm, 28 µm, 29 µm, 30 µm, 31 µm, 32 µm, 33 µm, 34 µm, 35 µm, 36 µm, 37 µm, 38 µm, 39 µm, 40 µm, 41 µm, 42 µm, 43 µm, 44 µm, 45 µm, 46 µm, 47 µm, 48 µm, 49 µm, 50 µm or any value between any two of the values, preferably no more than 20 µm. The co-processed excipient is spherical particles, and the suitable particle size (such as D90 value) of the active ingredient enables an easy and uniform mixing with the excipient, so that the pharmaceutical composition can have better *in vitro* dissolution behavior and superior content uniformity.

The method for obtaining the particles of the particle size is as follows: (3a*R*,5*s*,6a*S*)-*N*-(3-methoxy-1,2,4-thiadiazol-5-yl)-5-(methyl(7*H*-pyrrolo[2,3-*d*]pyrimidin-4-yl) amino)hexahydrocyclopenta[*c*]pyrrole-2(1*H*)-carboxamide or a pharmaceutically acceptable salt thereof with a large particle size is pulverized by micronization to a desired particle size using a chopper, a hammer mill, a cryogenic pulverizer, a jet mill or the like with the temperature controlled during the pulverization. (3a*R*,5*s*,6a*S*)-*N*-(3-methoxy-1,2,4-thiadiazol-5-yl)-5-(methyl(7*H*-pyrrolo[2,3-*d*]pyrimidin-4-yl) amino)hexahydrocyclopenta[*c*]pyrrole-2(1*H*)-carboxamide or a pharmaceutically acceptable salt thereof with a large particle size can also be pulverized together with necessary excipients, so that a pharmaceutical mixture with better uniformity can be obtained while the particle size of the particles is ensured.

Further, after the pharmaceutical composition of the disclosure is stored under high humidity conditions (25 °C/90% RH) for 30 days, the dissolution of the active ingredient is more than 85% (e.g., 85%, 86%, 87%, 88%, 89%, 90%, 91%, 92%, 93%, 94%, 95%, 97% or more) at 15 minutes or more than 90% (e.g., 90%, 91%, 92%, 93%, 94%, 95%, 97% or more) at 30 minutes, when tested in a dissolution test at a paddle speed of 50 rpm at 37 ± 0.5 °C according to the Dissolution Test Method 2 (paddle method) of Appendix, Chinese Pharmacopoeia, Volume II, 2015 Edition, using a phosphate solution at pH 6.8 as a dissolution medium.

In another aspect, after the pharmaceutical composition of the disclosure is stored under long-term conditions (30 ± 2 °C and RH 65% ± 5%) for 1-12 months, the dissolution of the active ingredient is more than 90% (e.g., 90%, 91%, 92%, 93%, 94%, 95%, 97% or more) at 30 minutes, when tested in a dissolution test at a paddle speed of 50 rpm at 37 ± 0.5 °C according to the Dissolution Test Method 2 (paddle method) of Appendix, Chinese Pharmacopoeia, Volume II, 2015 Edition, using a phosphate solution at pH 6.8 as a dissolution medium.

In another aspect, after the pharmaceutical composition of the disclosure is stored under accelerated conditions (40 ± 2 °C and RH 75% ± 5%) for 1-3 months, the dissolution of the active ingredient is more than 90% (e.g., 90%, 91%, 92%, 93%, 94%, 95%, 97% or more) at 30 minutes, when tested in a dissolution test at a paddle speed of 50 rpm at 37 ± 0.5 °C according to the Dissolution Test Method 2 (paddle method) of Appendix, Chinese Pharmacopoeia, Volume II, 2015 Edition, using a phosphate solution at pH 6.8 as a dissolution medium.

In some embodiments, the pharmaceutically acceptable salt of the active ingredient (3a*R*,5*s*,6a*S*)-*N*-(3-methoxy-1,2,4-thiadiazol-5-yl)-5-(methyl(7*H*-pyrrolo[2,3-*d*]pyrimidin-4-yl) amino)hexahydrocyclopenta[*c*]pyrrole-2(1*H*)-carboxamide of the disclosure is selected from the group consisting of a hydrosulfate salt.

In another aspect, the pharmaceutical composition of the disclosure is further coated where necessary, for example, coated with an enteric coating to meet enteric release requirements. The disclosure also provides a method for preparing the above pharmaceutical composition, which comprises: a) mixing (3a*R*,5*s*,6a*S*)-*N*-(3-methoxy-1,2,4-thiadiazol-5-yl)-5-(methyl(7*H*-pyrrolo[2,3-*d*]pyrimidin-4-yl) amino)hexahydrocyclopenta[*c*]pyrrole-2(1*H*)-carboxamide or a pharmaceutically acceptable salt thereof with a filler and optionally at least one excipient from a disintegrant and a lubricant; b) granulating the mixture obtained from a), followed by tableting or direct tableting. In another aspect, the disclosure also provides use of the above pharmaceutical composition in preparing a medicament for treating a JAK kinase-associated disease, preferably rheumatic and rheumatoid arthritis.

In another aspect, the disclosure also provides a method of the above pharmaceutical composition for treating a JAK kinase-associated disease, preferably rheumatic and rheumatoid arthritis.

The "composition" of the disclosure refers to a mixture comprising one or more of the active ingredients described herein and other chemical components, for example, physiological/pharmaceutically acceptable carriers and excipients. The purpose of the pharmaceutical composition is to promote the administration to an organism, which facilitates the absorption of the active ingredient, thereby exerting biological activities. As used herein, a "composition" and a "formulation" are not mutually exclusive.

The "total weight of the pharmaceutical composition" described in the disclosure is the numerical range of the amount of the active ingredient or other pharmaceutical excipients calculated based on the weight of the tablet core without the coating agent.

The "free of" described in the disclosure means that it is within the scope of "substantially free of" as would be acceptable to one of ordinary skill in the art, i.e., the metal element in the pharmaceutical composition is not sufficient to affect the stability of the composition after being stored for a period of time.

The "about" described in the disclosure means that it is within an acceptable error range for a particular value as determined by one of ordinary skill in the art, which will depend in part on how the value is measured or determined, i.e., the limitations of the measurement system. In the context of a particular assay, result or embodiment, "about" means that it is within one standard deviation or a range of up to 5% according to the practice in the art, unless otherwise explicitly stated in the example or elsewhere in the specification. In another aspect, the values in the disclosure are instrument measurements, and have a certain degree of error. Generally, ± 10% falls within a reasonable error range. Of course, the context in which the value is used needs to be taken into account, for example, for the particle size of the active ingredient, the error range of the value after the measurement shall no exceed ± 10%, and may be ± 9%, ± 8%, ± 7%, ± 6%, ± 5%, ± 4%, ± 3%, ± 2% or ± 1%, preferably ± 5%.

### DETAILED DESCRIPTION

The disclosure is further illustrated in detail by the following examples and experimental examples. These examples and experimental examples are for illustrative purposes only and are not intended to limit the scope of the disclosure. Experimental procedures without specific conditions indicated in the examples of the disclosure are generally conducted according to conventional conditions favorable to production or according to conditions recommended by the manufacturers of the raw materials or commercial products. Reagents without specific sources indicated are conventional reagents purchased from the market.

In the following examples, compound A was used to indicate a hydrosulfate salt of (3a*R*,5*s*,6a*S*)-*N*-(3-methoxy-1,2,4-thiadiazol-5-yl)-5-(methyl(7*H*-pyrrolo[2,3-*d*]pyrimidin-4-yl) amino)hexahydrocyclopenta[*c*]pyrrole-2(1*H*)-carboxamide.

### Example 1

According to the formula proportions of Table 1, compound A, lactose, microcrystalline cellulose and croscarmellose sodium were mixed well, and the mixture was subjected to wet granulation using a 3% hydroxypropyl methylcellulose (HPMC E5) aqueous solution as a wetting agent, followed by tableting to obtain a tablet.

**Table 1**

| Ingredient | Formula 1/mg |
|---|---|
| Compound A | 1.24 |
| Lactose | 70.56 |
| Microcrystalline cellulose | 22.00 |
| Croscarmellose sodium | 4.00 |
| HPMC E5 | 1.20 |
| Purified water | 38.00 |
| Magnesium stearate | 1.00 |
| Total | 100.00 |

The tablet prepared according to above formula was stored under conditions of 25 °C and RH 90% for 30 days, and determined for the dissolution according to the Dissolution Test Method 2 (paddle method) of Appendix, Chinese Pharmacopoeia, Volume II, 2010 Edition or according to the Method 2 (paddle method) of General Chapter 0931, Chinese Pharmacopoeia, Volume IV, 2015 Edition.

Dissolution test conditions: tested at a paddle speed of 50 rpm at 37 ± 0.5 °C using 500 mL of a 0.1 mol/L hydrochloric acid solution as a dissolution medium. The specific data for dissolution are shown in Table 2.

**Table 2**

| Time (min) | Day 0 | Day 30 |
|---|---|---|
| | Dissolution (%) | |
| 30 | 98.5 | 93.9 |

Dissolution test conditions: tested at a paddle speed of 50 rpm at 37 ± 0.5 °C using 500 mL of a hydrochloric acid buffer at pH 6.8 as a dissolution medium. The specific data for dissolution are shown in Table 3.

**Table 3**

| Time (min) | Day 0 | Day 30 |
|---|---|---|
| | Dissolution (%) | |
| 30 | 94.7 | 9.7 |

Conclusion: 1) the dissolution of the tablet prepared according to the formula 1 at 30 minutes varies in different dissolution media, suggesting that the 0.1 mol/L hydrochloric acid medium cannot truly reflect the changes in the dissolution of the sample, while the phosphate medium at pH 6.8 can reflect the real dissolution of the sample;
2) after being stored under high humidity conditions for 30 days, the dissolution of the tablet in the phosphate medium at pH 6.8 is significantly slowed down and the dissolution is incomplete, although the related substances of the sample are not changed, therefore, the formulation product has quality risk in the marketing.

### Example 2

According to the formula proportions of Table 4, compound A, lactose, microcrystalline cellulose and low-substituted hydroxypropylcellulose were mixed well, and the mixture was subjected to wet granulation using a 3% hydroxypropyl methylcellulose aqueous solution as a wetting agent, followed by tableting to obtain a tablet.

**Table 4**

| Ingredient | Formula 2/mg |
|---|---|
| Compound A | 1.24 |
| Lactose | 70.56 |
| Microcrystalline cellulose | 22.00 |
| Low-substituted hydroxypropylcellulose | 8.00 |
| HPMC E5 | 1.20 |
| Purified water | 38.80 |
| Magnesium stearate | 1.00 |
| Total | 100 |

### Dissolution Test

The tablet prepared according to above formula was stored under conditions of 25 °C and RH 90% for 30 days, and determined for the dissolution according to the Method 2 (paddle method) of General Chapter 0931, Chinese Pharmacopoeia, Volume IV, 2015 Edition. Dissolution test conditions: tested at a paddle speed of 50 rpm at 37 ± 0.5 °C using 500 mL of a hydrochloric acid buffer at pH 6.8 as a dissolution medium. The specific data for dissolution are shown in Table 5.

**Table 5**

| Time (min) | Day 0 | Day 30 |
|---|---|---|
| | Dissolution (%) | |
| 30 | 95.7 | 45.4 |

Conclusion: 1) the dissolution of the tablets prepared by the formula 1 and formula 2 is rapid and complete initially, and after being stored for a period of time under high humidity conditions, the disintegration rate of the tablets is reduced to different degrees, which leads to a decrease in the dissolution rate and incomplete dissolution;
2) compared with the formula 1, the formulation formula including the nonmetallic disintegrant and low-substituted hydroxypropylcellulose, can improve the solubility of the pharmaceutical composition, particularly the dissolution of a sample after being stored at a high temperature for a period of time, and it is speculated that the metal ions will complex with the active ingredient, which affects the disintegration rate of the pharmaceutical composition, so that the dissolution of the pharmaceutical composition after being stored at the high temperature is affected.

### Example 3

According to the proportions of Table 6, compound A, lactose, cellulose-lactose C80 and silicon dioxide were mixed well, followed by the addition of stearic acid, and then the mixture was mixed well. The resulting material was subjected to direct tableting to obtain the desired tablet.

**Table 6**

| Ingredient | Formula 3/mg |
|---|---|
| Compound A | 4.95 |
| Lactose | 109.05 |
| Cellulose-lactose C80 | 80.00 |
| Silicon dioxide | 2.00 |
| Stearic acid | 4.00 |
| Total | 200 |

### Dissolution Test

The tablet prepared according to above formula was stored under conditions of 25 °C and RH 90% for 30 days, and determined for the dissolution according to the Method 2 (paddle method) of General Chapter 0931, Chinese Pharmacopoeia, Volume IV, 2015 Edition. Dissolution test conditions: tested at a paddle speed of 50 rpm at 37 ± 0.5 °C using 1000 mL of a hydrochloric acid buffer at pH 6.8 as a dissolution medium. The specific data for dissolution are shown in Table 7.

**Table 7**

| Time (min) | Day 0 | Day 30 |
|---|---|---|
| | Dissolution (%) | |
| 30 | 99.0 | 86.8 |

Conclusion: after being stored under high humidity conditions, the dissolution of the tablet prepared according to the formula 3 at 30 minutes is reduced, but can still reach above 85% as compared to that of the tablet prepared according to the formula 2, therefore, the product quality requirement is met.

### Example 4

According to the proportions of Table 8, compound A, lactose, cellulose-lactose C80 and colloidal silicon dioxide were mixed well, followed by the addition of stearic acid, and then the mixture was mixed well. The resulting material was subjected to direct tableting to obtain the desired tablet.

**Table 8**

| Ingredient | Formula 4/mg |
|---|---|
| Compound A | 0.618 |
| Lactose | 50.182 |
| Cellulose-lactose C80 | 40.00 |
| Low-substituted | 5.00 |
| hydroxypropylcellulose | |
| Silicon dioxide | 1.30 |
| Stearic acid | 2.00 |
| Total | 100 |

### Dissolution Test

The tablet prepared according to above formula was stored under conditions of 25 °C and RH 90% for 30 days, and determined for the dissolution according to the Method 2 (paddle method) of General Chapter 0931, Chinese Pharmacopoeia, Volume IV, 2015 Edition. Dissolution test conditions: tested at a paddle speed of 50 rpm at 37 ± 0.5 °C using 500 mL of a hydrochloric acid buffer at pH 6.8 as a dissolution medium. The specific data for dissolution are shown in Table 9.

**Table 9**

| Time (min) | Day 0 | Day 30 |
|---|---|---|
| | Dissolution (%) | |
| 5 | 96 | 79 |
| 10 | 100 | 91 |
| 15 | 101 | 96 |
| 30 | 101 | 101 |

It can be seen that the above sample maintains excellent dissolution after being stored under high humidity conditions for a period of time, such as 30 days, and it is expected that the formulation product meets the requirement of controllable quality risk for the product after marketing.

### Example 5

According to the proportions of Table 10, compound A, lactose, cellulose-lactose C80, low-substituted hydroxypropylcellulose, colloidal silicon dioxide and stearic acid were mixed well. The resulting material was subjected to direct tableting to obtain the desired tablet. The tablet was coated with a film coating premix (gastric soluble), and packaged with pharmaceutical aluminum foil.

**Table 10**

| Ingredient | Formula/mg |
|---|---|
| Compound A | 9.89 |
| Lactose | 196.91 |
| Cellulose-lactose C80 | 160.00 |
| Low-substituted hydroxypropylcellulose | 20.00 |
| Silicon dioxide | 5.20 |
| Stearic acid | 8.00 |

The above sample was stored under conditions of 30 ± 2 °C, RH 65% ± 5% (long-term) and 40 ± 2 °C, RH 75% ± 5% (accelerated) to investigate the stability and dissolution of the sample. Dissolution test conditions: tested at a paddle speed of 50 rpm at 37 ± 0.5 °C using 500 mL of a hydrochloric acid buffer at pH 6.8 as a dissolution medium.

**Table 11**

| Time (month) | Long-term | | Accelerated | |
|---|---|---|---|---|
| | Content (%) | Dissolution (%)^{b} | Content (%) | Dissolution (%)^{b} |
| 0 | 98.3 | 94-97 | 98.3 | 94-97 |
| 1 | / | / | 98.7 | 97-101 |
| 2 | / | / | 98.2 | 95-98 |
| 3 | 97.5 | 95-98 | 97.7 | 95-97 |
| 6 | 97.2 | 96-99 | 97.4 | 97-99 |
| 9 | 97.6 | 95-98 | / | / |
| 12 | 96.2 | 95-97 | / | / |

| | | | | |
|---|---|---|---|---|
| Note: a. calibrating the content of the sample by HPLC; b. dissolution after 30 minutes. | | | | |

## Claims

1. A pharmaceutical composition, comprising an active ingredient (3a*R*,5*s*,6a*S*)-*N*-(3-methoxy-1,2,4-thiadiazol-5-yl)-5-(methyl(7*H*-pyrrolo[2,3-*d*]pyrimidin-4-yl) amino)hexahydrocyclopenta[*c*]pyrrole-2(1*H*)-carboxamide or a pharmaceutically acceptable salt thereof and a filler, wherein the filler is selected from the group consisting of a co-processed excipient, and the co-processed excipient is preferably microcrystalline cellulose-lactose, cellulose-lactose or corn starch-lactose, and more preferably microcrystalline cellulose-lactose or cellulose-lactose.

2. The pharmaceutical composition according to claim 1, wherein the filler, based on the total weight of the pharmaceutical composition, has a content of 40%-95%, preferably 60%-92%.

3. The pharmaceutical composition according to claim 1 or 2, further comprising a disintegrant, wherein the disintegrant, based on the total weight of the pharmaceutical composition, has a content of 1%-30%, preferably 2%-20%.

4. The pharmaceutical composition according to claim 3, wherein the disintegrant is free of a metal element, and is preferably at least one of pre-gelatinized starch, adipic acid, alginic acid, gelatinized starch, crospolyvinylpyrrolidone and low-substituted hydroxypropylcellulose.

5. The pharmaceutical composition according to any one of claims 1-4, further comprising at least one of a glidant and a lubricant, wherein the glidant or the lubricant, based on the total weight of the pharmaceutical composition, has a content of 0.5%-5%, preferably 1%-5%.

6. The pharmaceutical composition according to claim 5, wherein the glidant is free of a metal element, and is preferably at least one of silicon dioxide, light anhydrous silicic acid, crystalline cellulose, stearic acid and corn starch, and more preferably silicon dioxide; and the lubricant is free of a metal element, and is preferably at least one of stearic acid, glyceryl behenate, hydrogenated vegetable oil and silicon dioxide.

7. The pharmaceutical composition according to any one of claims 1-6, being free of a metal element, wherein the metal element is preferably an alkali metal or an alkaline earth metal.

8. The pharmaceutical composition according to any one of claims 1-7, wherein the filler further comprises lactose.

9. The pharmaceutical composition according to claim 8, wherein the co-processed excipient and lactose are in a weight ratio of 1:2-5:1, preferably 1:2-2:1.

10. The pharmaceutical composition according to claims 1-9, comprising the following ingredients based on the total weight of the pharmaceutical composition:
1) 0.1%-30% (3a*R*,5*s*,6a*S*)-*N*-(3-methoxy-1,2,4-thiadiazol-5-yl)-5-(methyl(7*H*-pyrrolo[2,3-*d*]pyrimidin-4-yl) amino)hexahydrocyclopenta[*c*]pyrrole-2(1*H*)-carboxamide or a pharmaceutically acceptable salt thereof,
2) 40%-95% filler, wherein the co-processed excipient and the lactose are in a weight ratio of 1:2-5:1,
3) optionally 1%-30% disintegrant,
4) optionally 0.5%-5% glidant, and
5) optionally 0.5%-5% lubricant,
wherein further, the pharmaceutical composition is preferably free of a metal element.

11. A pharmaceutical composition comprising (3a*R*,5*s*,6a*S*)-*N*-(3-methoxy-1,2,4-thiadiazol-5-yl)-5-(methyl(7*H*-pyrrolo[2,3-*d*]pyrimidin-4-yl) amino)hexahydrocyclopenta[*c*]pyrrole-2(1*H*)-carboxamide or a pharmaceutically acceptable salt thereof, wherein the pharmaceutical composition is free of an excipient containing a metal element.

12. The pharmaceutical composition according to claim 11, further comprising a filler, wherein the filler is preferably at least one of lactose, microcrystalline cellulose-lactose, cellulose-lactose and corn starch-lactose.

13. The pharmaceutical composition according to claim 12, further comprising at least one of a disintegrant, a glidant and a lubricant.

14. The pharmaceutical composition according to any one of claims 11-13, comprising the following ingredients based on the total weight of the pharmaceutical composition:
1) 0.1%-30% (3a*R*,5*s*,6a*S*)-*N*-(3-methoxy-1,2,4-thiadiazol-5-yl)-5-(methyl(7*H*-pyrrolo[2,3-*d*]pyrimidin-4-yl) amino)hexahydrocyclopenta[*c*]pyrrole-2(1*H*)-carboxamide or a pharmaceutically acceptable salt thereof,
2) 40%-95% filler, wherein the filler is preferably at least one of lactose, microcrystalline cellulose-lactose, cellulose-lactose and corn starch-lactose,
3) optionally 1%-30% disintegrant,
4) optionally 0.5%-5% glidant, and
5) optionally 0.5%-5% lubricant,
wherein further, the pharmaceutical composition is preferably free of an excipient containing a metal element.

15. The pharmaceutical composition according to any one of claims 1-14, wherein the pharmaceutically acceptable salt of the active ingredient is selected from the group consisting of a hydrogen sulphate.

16. The pharmaceutical composition according to any one of claims 1-15, wherein after the pharmaceutical composition is stored under high humidity conditions for 30 days, the dissolution of the active ingredient is more than 85% at 15 minutes, preferably more than 90% at 30 minutes, when tested in a dissolution test at a paddle speed of 50 rpm at 37 ± 0.5 °C using a phosphate solution at pH 6.8 as a dissolution medium.

17. A method for preparing the pharmaceutical composition according to any one of claims 1-16, comprising: a) mixing (3a*R*,5*s*,6a*S*)-*N*-(3-methoxy-1,2,4-thiadiazol-5-yl)-5-(methyl(7*H*-pyrrolo[2,3-*d*]pyrimidin-4-yl) amino)hexahydrocyclopenta[*c*]pyrrole-2(1*H*)-carboxamide or a pharmaceutically acceptable salt thereof with a filler and optionally at least one excipient from a disintegrant and a lubricant; b) granulating the mixture obtained from a), followed by tableting or direct tableting.

18. Use of the pharmaceutical composition according to any one of claims 1-16 in preparing a medicament for treating a JAK kinase-associated disease, preferably rheumatic and rheumatoid arthritis.
